# EUROPÄISCHE PATENTSCHRIFT

(11) **EP 3 003 033 B1**
(45) Veröffentlichungstag und Bekanntmachung des Hinweises auf die Patenterteilung: **10.10.2018**
(21) Anmeldenummer: 14726520.1
(22) Anmeldetag: 20.05.2014
(51) Int. Cl.: A01N 25/30

(54) **FLUORTENSIDE IN PESTIZIDEN**
FLUOROSURFACTANTS IN PESTICIDES
TENSIO-ACTIFS FLUORÉS DANS DES PESTICIDES

(30) Priorität: 04.06.2013 EP 13002875
(43) Veröffentlichungstag der Anmeldung: 13.04.2016
(73) Patentinhaber: Merck Patent GmbH, 64293 Darmstadt (DE)
(72) Erfinder: PAHNKE, Joerg, 64285 Darmstadt (DE); JONSCHKER, Gerhard, 64646 Heppenheim (DE); SCHELLENBERGER, Steffen, 64293 Darmstadt (DE)
(86) Internationale Anmeldenummer: PCT/EP2014/001354
(87) Internationale Veröffentlichungsnummer: WO 2014/194984

(56) Entgegenhaltungen:
- WO-A1-2012/084118
- WO-A2-2010/003567
- DE-A1-102009 030 846
- US-A- 5 198 467
- DATABASE CAPLUS, [Online] 22. Januar 2004 (2004-01-22), NAGAI TAKAFUMI ET AL: "Perfluoropolyether-containing amphiphilic compounds and their uses", XP002714401, gefunden im CAPLUS; STN Database accession no. 2004-52783 & JP 2004 018394 A (DAIKIN IND LTD) 22. Januar 2004 (2004-01-22)
- Jan Alexander ET AL: "Perfluorooctane sulfonate (PFOS), perfluorooctanoic acid (PFOA) and their salts Scientific Opinion of the Panel on Contaminants in the Food chain 1 (Question N o EFSA-Q-2004-163) Adopted on 21 February 2008 PANEL MEMBERS", The EFSA Journal, 1. Januar 2008 (2008-01-01), Seiten 1-131, XP055130898, Gefunden im Internet: URL:http://www.efsa.europa.eu/en/efsajourn al/doc/653.pdf [gefunden am 2014-07-22]

## Beschreibung

Die vorliegende Erfindung betrifft die Verwendung von Fluortensiden der Formeln (III-1) und/oder (III-2) in Pestiziden.

Für die Herstellung von Pestiziden werden, insbesondere im Pflanzenschutz, neben den regulären Wirkstoffen sogenannte Adjuvantien eingesetzt. Adjuvantien sind hierbei nach ASTM E 1519 Stoffe, die den Pestizidwirkstoffen zugesetzt werden, um die Wirkung von Pestiziden und/oder deren physikalische Eigenschaften zu verbessern. Adjuvantien werden in zwei wesentliche Funktionsklassen unterteilt:
A. Adjuvantien, die die Wirksamkeit des Pestizids erhalten oder verbessern. Hierunter fallen vor allem Stoffe, die zu folgenden Effekten führen: verbesserte Benetzungseigenschaften (z.B. Superspreitung), effizientere Adsorption und Aufnahme des Wirkstoffs (Penetration), befeuchtende Wirkung, Tröpfchengröße als Funktion vom Spritzdruck etc.
B. Adjuvantien, die die praktische Verwendung beeinflussen. Hierbei sind in erster Linie zu nennen: Emulgatoren, pH-Modifikatoren, Schaumbildner, Entschäumer, Substanzen zur Reduktion des Sprühüberschusses (Drift).

Tenside sind eine der wichtigsten Klassen innerhalb der Adjuvantien und kommen in beiden Anwendungsfeldern zum Einsatz. Sie erfüllen dabei in erster Linie die Funktionen zur verbesserten Benetzung der Oberfläche und gewährleisten dadurch eine effizientere Penetration des Wirkstoffs. Weiterhin fungieren sie als Löslichkeitsvermittler in der Form von Emulgatoren und Dispergieradditiven, um die zumeist unpolaren Wirkstoffe in wässrigen Lösungen zu homogenisieren. Als Emulgatoren werden sie in verschiedenen Formulierungen eingesetzt: Spritzpulver (WP), Öl in Wasser- bzw. Wasser in Öl-Emulsionen (EW bzw. EO), Suspensionen (SC), Suspoemulsionen (SE), emulgierbare Konzentrate (EC) oder auch Granulate bzw. wasserdispergierbare Granulate.

Da Pflanzenoberflächen häufig durch eine epikutikuläre hydrophobe Wachsschicht gekennzeichnet sind sowie verschiedene Blattmorphologien (z.B. Haare, Wachskristalle) ausprägen, werden sie durch wässrige Wirkstofflösungen nur schlecht benetzt. Hierdurch wird die Aufnahme des Wirkstoffs durch die Pflanze durch zwei wesentliche Sachverhalte stark eingeschränkt: Zum einen kommt es während des Sprühprozesses zu einem "Abperlen" der wässrigen Wirkstofflösung, so dass nicht genügend Kontaktzeit mit der Blattoberfläche besteht, um den Wirkstoff aufzunehmen. Zum anderen bilden die anhaftenden Tropfen der Sprühlösung nur sehr kleine Kontaktflächen auf der Blattoberfläche aus, wodurch die Aufnahmekinetik des Wirkstoffes eingeschränkt wird. Durch die Zugabe von Tensiden können die Benetzungseigenschaften veränderte werden und so die Effizienz stark verbessert werden. Im optimalen Fall leistet das Tensid eine starke Reduzierung der Oberflächenspannung um die mit Wachs belegte Pflanzenoberfläche zu benetzen und zeigt darüber hinaus superspreitendes Benetzungsverhalten. Adjuvantien, ihre Einteilung, Eigenschaften und Wirkweisen sind in Winnacker-Küchler, "Chemische Technologie", Band 7, C. Hanser-Verlag München, 4. Auflage 1986, beschrieben.

Bisher wurden hauptsächlich zwei Materialklassen von Tensiden bzw. Tensidmischungen als Adjuvantien eingesetzt: Tenside basierend auf Kohlenwasserstoffen und Siloxanen. So wird im Patent CA 2230769 der Einsatz von nichtionischen Siloxantensiden beschrieben, der zu einer größeren Benetzungsfläche der Sprühlösung und damit zu einer effizienteren Wirksamkeit des eingesetzten Pestizids führt. Weiterhin wird in WO 2010/003889 die erhöhte Effizienz von Wirkstoffen basierend auf der Tensidzugabe von ethoxylierten Sorbitolen eingegangen. Weitere Verwendungen von Tensiden als Adjuvantien werden unter anderem in Conference Proceedings 9th International Symposium on Adjuvants for Agrochemicals, ISAA 2010 beschrieben.

Bisherige Tenside als Adjuvantien, insbesondere nichtionische Silikon,- und Kohlenwasserstofftenside, zeigen nur unzureichende Benetzungseigenschaften auf für den Pestizideinsatz relevanten Oberflächen, da ihr Potential zur Reduzierung der Oberflächenspannung in Wasser auf > 20 mN/m beschränkt ist. Darüber hinaus zeigen nur wenige dieser Tenside superspreitende Eigenschaften und neigen zu einer erhöhten Schaumbildung in Pestizidformulierungen, die aufgrund der schlechteren Verarbeitung unerwünscht ist. Klassische Fluortenside basieren zudem auf langkettigen perfluorierten Ketten, die sich als hoch bioakkumulativ und toxisch herausgestellt haben und beim Versprühen inhalationstoxisch wirken, so dass umfangreiche Schutzmaßnahmen des Bedienpersonals notwendig werden.

Fluortenside, die als Adjuvantien in Pestiziden verwandt werden, sind in der Literatur nur wenig detailliert beschrieben. M. Pisante et al. (J. Pestic. Sci., 32(1), 2007, 16-23) gehen auf erkennbare Vorteile hinsichtlich Benetzung und die damit verbundene effizientere Wirkstoffaufnahme ein. US 5,198,467 offenbart pestizide Zusammensetzungen enthaltend ein anionisches lineares Perfluoralkylsulfonat und ein Insektizid,

Spezielle Anwendungen von Sulfosuccinaten und/oder Sulfotricarballylaten mit verschiedenen fluorierten Seitenketten sind in US 4,968,599 und US 4,988,610 sowie US 6,890,608 beschrieben und in A.R. Pitt et al., Colloids and Surfaces A: Physicochemical and Engineering Aspects, 1996, 114, 321-335; A.R. Pitt, Progr. Colloid Polym. Sci, 1997, 103, 307-317 und Z.-T. Liu et al., Ind. Eng. Chem. Res. 2007, 46, 22-28. Weitere Fluortenside, insbesondere Succinate und Tricarballylate mit fluorierten Alkylgruppen, sind in WO 2009/149807, WO 2010/003567, WO 2010/149262, WO 2011/082770 und WO 2012/084118 beschrieben.

Es wurde nun gefunden, dass durch die Verwendung von bestimmten Fluortensiden als Adjuvantien in Pestiziden die Nachteile des Standes der Technik vermieden werden. Pestizide umfassen hierbei sowohl Schädlingsbekämpfungsmittel allgemein als auch Pflanzenschutzmittel. Insbesondere in Pflanzenschutzmitteln ist die Verwendung der erfindungsgemäßen Verbindungen vorteilhaft. Als Pestizide bzw. Pflanzenschutzmittel und Schädlingsbekämpfungsmittel werden in der vorliegenden Erfindung Formulierungen bezeichnet, die die entsprechenden Wirkstoffe zur Schädlingsbekämpfung bzw. zum Pflanzenschutz und Additive und/oder Lösemittel enthalten.

Ein erster Gegenstand der vorliegenden Erfindung ist die Verwendung von Verbindungen der Formeln (III-1) und/oder (III-2) in Pestiziden:
wobei * den jeweiligen Anknüpfungspunkt angibt

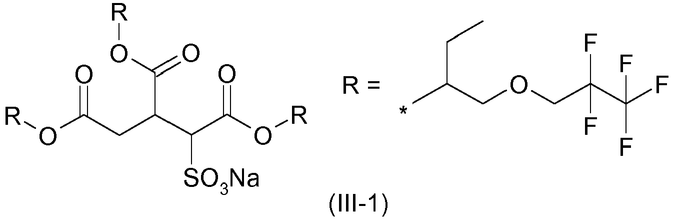

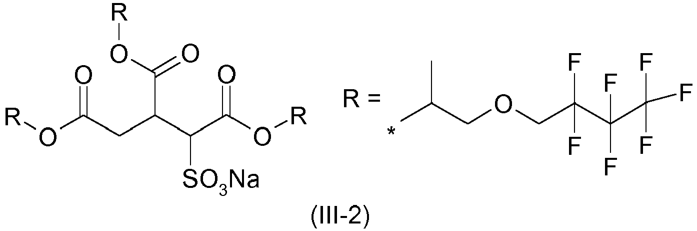

Die verwendeten Fluortenside der Formeln (III-1) und/oder (III-2) sind aus mehreren kurzkettigen Perfluoralkylgruppen aufgebaut. Die erfindungsgemäßen Verbindungen zeichnen sich durch verbesserte Eigenschaften im Einsatz als Adjuvantien aus.

Die erfindungsgemäßen verzweigten Fluortenside mit kurzkettigen Perfluoralkylketten können zu einem verbesserten ökotoxikologischen Profil führen, da solche Verbindungen nicht toxisch sind und keine Bioakkumulation sowie keine inhalative Toxizität zeigen. Damit entfallen persönliche Schutzmaßnahmen bei der Verarbeitung, die auf die Toxizität der Tenside zurückzuführen sind.

Bei ihrer Verwendung in Pestiziden, insbesondere in Pflanzenschutzmitteln, können die Verbindungen der Formeln (III-1) und/oder (III-2) sowohl die Wirksamkeit der Wirkstoffe verbessern und/oder auch z. B. als Dispergiermittel, Emulsionsstabilisator und/oder Schauminhibitor wirken.

Durch die Verwendung der erfindungsgemäßen Fluortenside können insbesondere die Benetzungseigenschaften von Pestiziden, insbesondere von Pflanzenschutzmitteln, deutlich verbessert werden. Mittels der Fluortenside der Formeln (III-1) und/oder (III-2) kann die Oberflächenspannung in Wasser unter 20 mN/m gesenkt werden, was im Vergleich zu den bisher eingesetzten Tensiden auf Basis von Siloxanen und Kohlenwasserstoffen zu einer deutlichen Verbesserung der Benetzungseigenschaften auf der Blattoberfläche führt.

Darüber hinaus zeigen die erfindungsgemäßen verzweigten Fluortenside superspreitende Benetzungseigenschaften. Dieses führt zu einer höheren Effizienz der verwendeten Wirkstoffe in den Pestiziden, da sowohl die Drift reduziert wird als auch die Kontaktfläche für die Aufnahme des Wirkstoffes erhöht wird. Durch die vergrößerte Oberfläche trocknet ein Sprühfilm des Pestizids auch schneller ein, dadurch konzentriert sich der aktive Wirkstoff homogen auf dem Blatt auf und kann nicht so schnell vom Blatt abtropfen.

Die hier beschriebenen Fluortenside verbessern auch zahlreiche weitere Eigenschaften. Somit kann im Ross-Miles Test sowie im Tego-Schaumtest die verminderte Schaumbildung in Wasser gezeigt werden. Dies ist insbesondere in der Herstellung von Sprühlösungen vorteilhaft.

Fluortenside gemäß Formeln (III-1) und/oder (III-2) können in allen Pestiziden, sowohl zum Pflanzenschutz wie auch allgemein zur Schädlingsbekämpfung, eingesetzt werden. Insbesondere in Pflanzenschutzmitteln können diese Verbindungen vorteilhaft eingesetzt werden, z. B. in Herbiziden, Insektiziden, Fungiziden, Algiziden, Aphiziden, Nematiziden, Akariziden, Molluskiziden, Bakteriziden, Viruziden, Rodentiziden oder Pflanzenwachstumsregulatoren. Die Verbindungen der Formeln (III-1) und/oder (III-2) sind auch zur Verwendung in weiteren Pflanzenschutzmitteln geeignet wie z. B. in Mitteln zur Veredlung von Gehölzen, Mitteln zur Verhütung von Wildschäden, Mitteln zur Bodenentseuchung und Beizmitteln zur Behandlung von Saat- und Pflanzgut.

Üblicherweise werden Verbindungen der Formeln (III-1) und/oder (III-2) flüssigen Pestizidformulierungen zugesetzt, die mittels Spritz- oder Sprühverfahren angewendet werden. Aber auch die Anwendung in anderen Pestizidformulierungen wie Spritzpulvern (WP), Öl in Wasser- bzw. Wasser in Öl-Emulsionen (EW bzw. EO), Suspensionen (SC), Suspoemulsionen (SE), emulgierbaren Konzentraten (EC) oder auch Granulaten bzw. wasserdispergierbaren Granulaten ist möglich. Besonders vorteilhaft ist die Anwendung der Verbindungen der Formeln (III-1) und/oder (III-2) in Formulierungen von Pflanzenschutzmitteln, die bei der Kultur von Nutz- oder Zierpflanzen über Spritz- oder Sprühverfahren aufgebracht werden.

Die erfindungsgemäßen Verbindungen gemäß den Formeln (III-1) und/oder (III-2) können auch als Isomerengemische (Konstitutions- und/oder Konfigurationsisomerengemische) vorliegen. Insbesondere Diastereomeren- und/oder Enantiomerengemische sind möglich.

Die erfindungsgemäßen Verbindungen der Formel (III-1) und/oder (III-2) können bevorzugt durch Veresterung von Aconitsäure bzw. dem Anhydrid oder Säurechlorid mit einem dem Alkoholteil der Formeln (III-1) oder (III-2) entsprechenden Alkohol und anschließende Addition an die Doppelbindung zur Einführung der Sulfonatgruppe hergestellt werden. Die erfindungsgemäßen Verbindungen können auch bevorzugt durch Veresterung von Zitronensäure mit einem dem Alkoholteil der Formel (III-1) oder (III-2) entsprechenden Alkohol und anschließende Funktionalisierung der Hydroxygruppen zur Einführung der Sulfonatgruppe hergestellt werden. Die verwendeten Alkohole sind kommerziell erhältlich und/oder ihre Herstellung ist dem Fachmann geläufig (z. B. DE 10 2009 030 846 A1; Heilmann et al. J. Fluorine Chem. 1992, 59, 387; Janulis et al. US 5,157,159 (1992); Carbohydrate Research 1991, 219, 33). Die Synthese erfindungsgemäßer Tricarballylate erfolgt bevorzugt in einer zweistufigen Synthese über die die entsprechenden Aconit- oder Zitronensäureester. Diese Synthesen sind in WO 2010/149262, WO 2011/082770 und WO 2012/084118 beschrieben.

Ein weiterer Gegenstand der Erfindung sind Pestizide enthaltend mindestens eine Verbindung der Formeln (III-1) und/oder (III-2) Erfindungsgemäße Pestizide können ein oder mehrere der genannten Fluortenside enthalten.
Bei den Pestiziden kann es sich sowohl um Schädlingsbekämpfungsmittel allgemein als auch um Pflanzenschutzmittel handeln, wie z. B. Herbizide, Insektizide, Fungizide, Algizide, Aphizide, Nematizide, Akarizide, Molluskizide, Bakterizide, Viruzide, Rodentizide, Pflanzenwachstumsregulatoren, Mittel zur Veredlung von Gehölzen, Mittel zur Verhütung von Wildschäden, Mittel zur Bodenentseuchung und Beizmittel zur Behandlung von Saat- und Pflanzgut.

Die Pestizide können in verschiedenen Formulierungen vorliegen, z. B. als Spritzpulver (WP), Öl in Wasser- bzw. Wasser in Öl-Emulsionen (EW bzw. EO), Suspensionen (SC), Suspoemulsionen (SE), emulgierbare Konzentrate (EC) oder auch Granulate bzw. wasserdispergierbare Granulate. Insbesondere Pflanzenschutzmitteln, die bei der Kultur von Nutz- oder Zierpflanzen über Spritz- oder Sprühverfahren aufgebracht werden, sind geeignet.

Des Weiteren können erfindungsgemäße Pestizide neben
den Fluortensiden der Formeln (III-1) und/oder (III-2) auch andere Tenside wie z.B. Silikontenside auf der Basis von Polydimethylsiloxanen, funktionelle Trisiloxane, Gemini-Kohlenwasserstofftenside sowie andere Kohlenwasserstofftenside enthalten. Die Verbindungen der Formeln (III-1) und/oder (III-2) können bevorzugt in Mischungen mit einer oder mehreren der Verbindungen der Formeln (VI) bis (X) verwendet werden.

Bevorzugt enthalten die Pestizide mindestens eine Verbindung der Formel (VI)

(RF-(Spacer)ₘ)ₙM (VI)

wobei
RF eine fluorhaltige Gruppe ist,
Spacer eine Einfachbindung oder eine organische funktionelle Kohlenstoffkette ist,
n≥1 ist,
m = 0-1 und
M eine anionische, kationische, amphotere oder nicht-ionische Gruppe ist.

Bevorzugte Verbindungen der Formel (VI) sind solche, in denen RF eine perfluorierte Alkylgruppe mit mindestens zwei C-Atomen, bevorzugt drei, insbesondere vier C-Atomen ist. Insbesondere bevorzugt sind Substanzen, die über eine perfluorierte C6-Kette verbunden mit einem Ethylrest verfügen.

Die Spacer Gruppe kann bevorzugt eine organische funktionelle Kohlenwasserstoffkette sein, z. B. ein lineares oder verzweigtes Alkylen, wobei ein oder mehrere nicht benachbarte C-Atome durch O, S, und/oder N ersetzt sein können.

Weiter bevorzugt sind Verbindungen der Formel (VI), in denen die Gruppierung (RF-(Spacer)ₘ)ₙ- gleich C_{n'}F_{2n'+r}, C_{n'}F₂ₙ₊₁-CH₂CH₂-, C_{n'}F_{2n'+1}-OCF₂CF₂-, C_{n'}F_{2n'+1}-OC₆H₄-, C_{n'}F_{2n'+1}-C(O)NH(CH₂)₃N=, C_{n'}F_{2n'+1}-SO₂NH(CH₂)₃N=, CF₃CCl₂(CF₂CFCl)ₙ₋₁-CF₂- oder C₈F₁₇CH₂CH₂Si(CH₃)₂- ist mit n' = 4-12. Besonders bevorzugt ist die Gruppierung C_{n'}F_{2n'+1}-CH₂CH₂- mit n' = 4-8.

Bevorzugte anionische Gruppen M sind -OPOO⁻, -COO⁻, -SO₃⁻, -OSO₃⁻, -OP(O)(O⁻)O- und -OP(O)O₂²⁻. Als Gegenionen werden hierbei bevorzugt H⁺, Na⁺, K⁺, Li⁺ oder NH₄⁺ verwendet.

Besonders bevorzugt sind Verbindungen der Formel (VI-a) 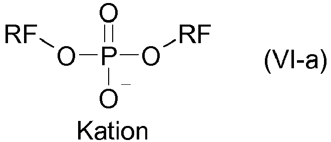 wobei RF = CF₃-(CF₂)ₙ-Spacer- ist, mit n = 0-12, und Kation = Na, K, Li, NH₄. Spacer hat die vorstehend angegebene Bedeutung.

Bevorzugte kationische Gruppen M sind -NR₃⁺ Gruppen mit R = C1-C4 Alkyl.

Bevorzugte amphotere Gruppen M sind -NR₂⁺-(CH₂)_{y}-COO⁻ Gruppen mit R = C1-C4 Alkyl und y = 1-3, bevorzugt y=1.

Bevorzugte nicht-ionische Gruppen M sind (OCH₂CH₂)ₙ-OR und -(OCH₂(CH₃)CH₂)ₙ-OR mit n = 4 - 40 und R = H oder C1-C4 Alkyl.

Bevorzugte Verbindungen der Formel (VI) sind insbesondere solche Verbindungen, in denen alle Variablen die bevorzugten Bedeutungen haben. Bevorzugt sind anionische Fluortenside z.B. auf der Basis von Phosphorsäure-, Carboxyl- und Sulfonsäuregruppen. Insbesondere bevorzugt sind Verbindungen der Formel (VI), in denen M eine anionische Gruppe ist und die Gruppierung (RF-(Spacer)ₘ)ₙ- gleich C_{n'}F_{2n'+1}-CH₂CH₂- mit n = 4-8 ist. Besonders bevorzugte Verbindungen sind hierbei Phosphorsäureester der Formel (VI-a), spezielle solche mit NH₄⁺ als Gegenion.

Die in den erfindungsgemäßen Pestiziden eingesetzten Verbindungen der Formel (VI) sind dem Fachmann bekannt. Sie können in Analogie zu bekannten Syntheseverfahren hergestellt werden oder sind kommerziell erhältlich. Die besonders bevorzugt verwendeten Phosphorsäureester sind z. B. unter dem Handelsnamen Chemguard® von der Firma Chemguard erhältlich, z. B. Chemguard® S760-P.

Bevorzugte Pestizide der Erfindung enthalten die vorgehend beschriebenen Verbindungen der Formeln (III-1) und/oder (III-2) und die vorgehend beschriebenen bevorzugten Verbindungen der Formel (VI).

Insbesondere bevorzugt sind Pestizide mit Fluortensiden der Formeln (III-1) und (III-2), in Kombination mit den genannten bevorzugten Phosphorsäureestern.

Die erfindungsgemäßen Pestizide können neben den Verbindungen der Formeln (III-1) und/oder (III-2) auch mindestens ein Sulfosuccinat, bevorzugt der Formel (VII), enthalten

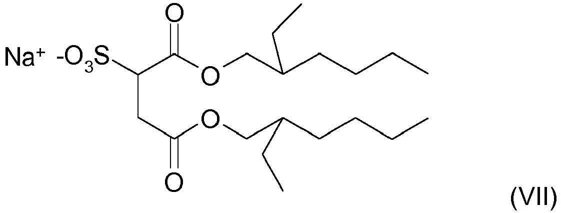

Die Pestizide können auch mindestens ein funktionelles Polymer auf der Basis von Polymethylsiloxan, bevorzugt der Formel (VIII), enthalten 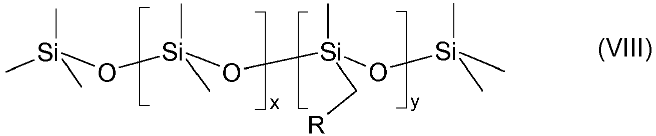 wobei x = 1-500, y = 1-500 und R = phenyl, methyl oder -(O-C₂H₃R')_{n"}-OR" ist, mit n" = 1-1000, R' = lineare und verzweigte Alkylrest und R" = lineare und verzeigte Alkylreste.

Die Pestizide können auch mindestens ein Trisiloxan-Derivat, bevorzugt der Formel (IX), enthalten

M₂D'(E_{n"'}P) (IX)

wobei M = (CH₃)₃SiO-, D' = Si(R"'), E = -OCH₂CH₂, n'" = 5 - 40 und P = -OH, -OMe, oder -OAc ist, mit R'" = lineare und/oder verzweigte Alkylkette.

Insbesondere bevorzugt ist die folgende Verbindung

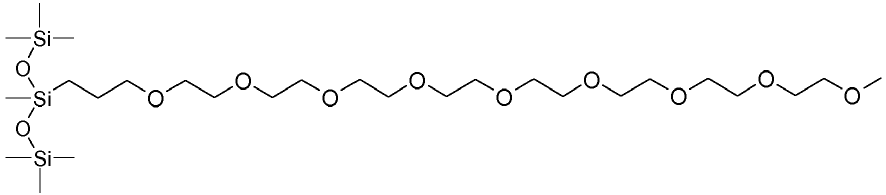

Die erfindungsgemäßen Pestizide können auch mindestens ein Gemini-Tensid enthalten, wobei zwei identische oder verschiedene amphiphile Gruppen, die aus Strukturen konventioneller Tenside aufgebaut sind, durch einen Spacer verbunden sind. Insbesondere bevorzugt sind z.B. Diacetylenderivate der Formel (X) 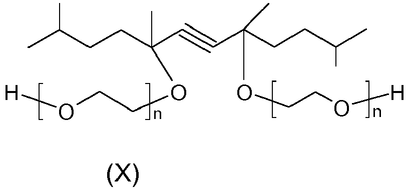 wobei n = 1-100 ist.

Wenn Mischungen der Verbindungen der Formeln (III-1) und/oder (III-2) mit anderen Tensiden verwendet werden, enthalten diese Mischungen eine oder mehrere der Verbindungen der Formeln (III-1) und/oder (III-2) und eine oder mehrere der Verbindungen der Formeln (II) bis (X) bevorzugt im Verhältnis von 70/30 bis 90/10, insbesondere im Verhältnis von 80/20 bis 85/15 (Gewichtsverhältnis).

Der Gehalt der Verbindungen der Formeln (III-1) und/oder (III-2) bzw. der Gehalt ihrer Mischungen, auch mit den genannten Tensiden der Formeln (VI)-(X), beträgt üblicherweise 0.01 - 1.0 Gew.-%, bevorzugt 0.05 - 0.5, insbesondere 0.05 - 0.2 Gew.-% bezogen auf die gesamte Pestizidformulierung. Besonders bevorzugt können 0.1 Gew.-%ige Formulierungen verwendet werden.

Pestizide, die die erfindungsgemäßen Verbindungen enthalten, können die dem Fachmann bekannten wasserlöslichen und/oder wasserunlöslichen Wirkstoffe enthalten, wie z. B. Glyphosat, Glufosinat, Paraquat, Hentazon, Fomesafen, Nicosulfuron, Chlorsulfuron, Butroxydim, Thifensulfuron, Aclonifen, Permethrin, Pyrethrin, Disulfoton, Armitraz, Diazinon, Metalazyl, u.a.
Neben den Verbindungen der Formeln (III-1) und/oder (III-2) bzw. Mischungen dieser Verbindungen mit Verbindungen der Formel (VI) und/oder Verbindungen der Formeln (VII) bis (X) können die erfindungsgemäßen Pflanzenschutzmittel auch übliche Lösemittel und/oder Additive wie z. B. Farbstoffe, Feuchthaltemittel, Rheologie modifizierende Mittel, Frostschutzmittel, etc. enthalten.
Die vollständigen Offenbarungen aller aufgeführten Anmeldungen und Veröffentlichungen sind durch Bezugnahme in diese Anmeldung eingeführt. In der Beschreibung und in den Beispielen gelten, soweit nicht anders angegeben, Prozentangaben als Gewichtsprozent. Die folgenden Beispiele erläutern die vorliegende Erfindung näher.

### Beispiele

### Beispiel 1: Synthese der Verbindung der Formel (III-1)

Gemäß der Patentanmeldung DE 10 2009 030 846 A1 wird der kettenverlängerte Alkohol aus den Edukten 2,2,3,3,3-Pentafluoropropan-1-ol (ABCR) und Butylencarbonat (TCI) hergestellt. Dieses Zwischenprodukt wird nach der beschriebenen Synthesevorschrift mit Aconitsäure (Alfa Aesar) verestert und dann im letzten Reaktionschritt mittels einer wässrigen Natriumhydrogensulfit-Lösung (Merck KGaA) sulfoniert. Die dynamische Oberflächenspannung wird nach der angegebenen Methode bestimmt und beträgt 28,2 mN/m (100 ms, 0,1 Gew.-%).

### Beispiel 2: Synthese der Verbindung der Formel (III-2)

Gemäß der Patentanmeldung DE 10 2009 030 846 wird der kettenverlängerte Alkohol aus den Edukten 2,2,3,3,4,4,4-Heptafluorobutan-1-ol (ABCR) und Propylencarbonat (Merck KGaA) hergestellt. Dieses Zwischenprodukt wird nach der beschriebenen Synthesevorschrift mit Aconitsäure verestert und dann im letzten Reaktionsschritt mittels einer wässrigen Natriumhydrogensulfit-Lösung sulfoniert. Die dynamische Oberflächenspannung wird nach der angegebenen Methode bestimmt und beträgt 66,6 mN/m (100 ms, 0,1 Gew.-%).

### Beispiel 3: Untersuchungen zur Benetzung auf PTFE-Folie nach ASTM E2044 8 (Standard Test Method for Spreading of Liquid Agricultural Spray Mixtures)

Gerät: Randwinkelmessgerät der Firma Krüss (DSA 100)
Substrat: PTFE-Folie der Firma Goodfellow GmbH; Messung auf Außenseite; (mittlere Rauigkeit R_{M} = 20-30nm)

### Eingesetzte Messmethode:

In Anlehnung an die ASTM E2044 wird der Tropfenbasisdurchmesser (d) einer wässrigen Tensidlösung (0,1 Gew% Tensidkonzentration) auf einer PTFE Folie nach verschiedenen Zeiten bestimmt. Dabei werden 20 µL der Flüssigkeit mit einer Spritze durch eine PTFE-Kapillare (Typ N44; d = 0,776mm) auf die entsprechende Oberfläche getropft (Abstand der Kapillare zur Oberfläche=5 mm). Mit Hilfe eines Randwinkel Messgerätes wird der Tropfenbasisdurchmesser nach 0 s; 10 s, 30 s und 60 s bestimmt. Der Tropfenbasisdurchmesser wird nach einer Videoaufnahme des Benetzungsvorgangs mit einem digitalen Lineal (Länge in Pixeln) ausgemessen und kann durch die Vergrößerung der Kapillare (der Durchmesser der Kapillare entspricht bei einer bestimmten Vergrößerung einer entsprechenden Pixelanzahl) auf eine Länge (in mm) umgerechnet werden.

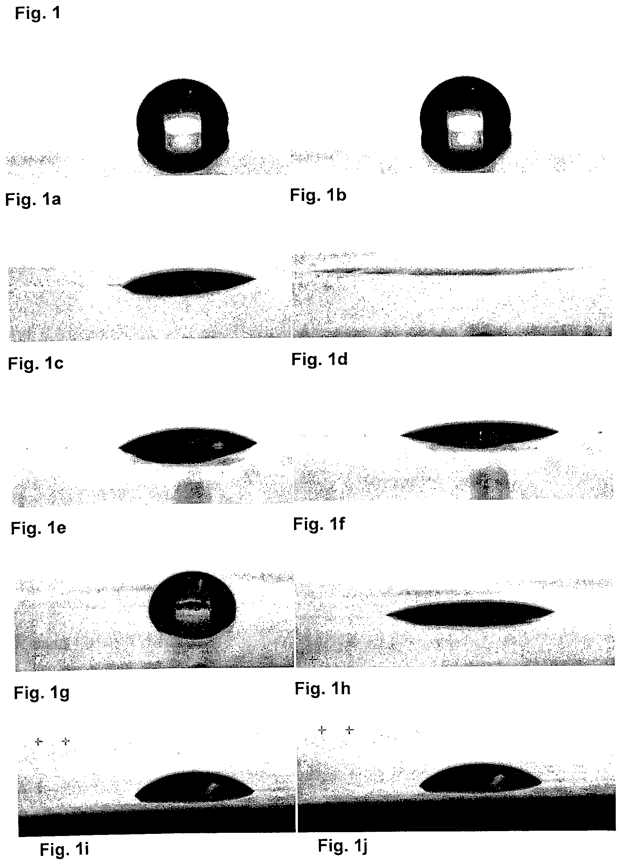

Abbildung 1 zeigt die Tropfen verschiedener wässriger Tensidlösungen im Vergleich zu reinem Wasser nach 0 s und nach 60 s. Dabei zeigen sich insbesondere für die Verbindung der Formel (III-2) deutliche Vorteile in Bezug auf das Benetzungsverhalten. Die Lösung der Verbindung der Formel (III-2) hat nach 60 s den größten Tropfenbasisdurchmesser und zeigt ein superspreitendes Verhalten auf Teflon.

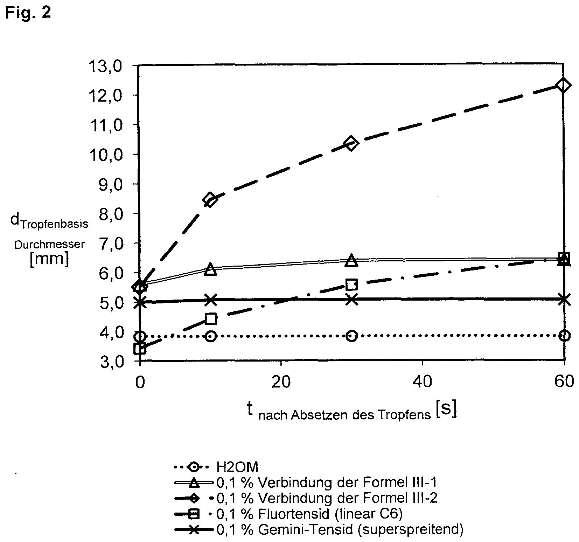

Abbildung 2 zeigt den Tropfenbasisdurchmesser in Abhängigkeit von verschiedenen Verweilzeiten (t) auf der PTFE Folie. Lösungen mit der Verbindung der Formel (III-2) und der Verbindung der Formel (III-1) zeigen sowohl nach 0 s als auch nach 10 s einen deutlich höheren Tropfenbasisdurchmesser als alle anderen Tensidlösungen. Dies ist wahrscheinlich mit einer sehr schnellen Benetzung der PTFE-Folie zu erklären.

### Beispiel 4: Untersuchungen zur Benetzung an Blättern der Weinrebe Müller-Thurgau (Vitis vinifera)

Gerät: Randwinkelmessgerät der Firma Krüss (DSA 100)
Substrat: Blätter der Weinrebe Müller-Thurgau; Blattausschnitt mit relativ homogener Oberfläche (Oberseite, Blattgröße∼7 x 8cm)

### Eingesetzte Messmethode:

Das Benetzungsverhalten von wässrigen Tensidlösungen (0,1% Tensidkonzentration) wird auf der Oberfläche von Weinpflanzenblättern mit einem Randwinkelmessgerät qualitativ untersucht. Hierfür wird ein homogener Blattausschnitt auf einem Uhrglas (d = 12 cm) mit konvexer Wölbung befestigt. Flüssigkeitstropfen können dadurch unabhängig den sonstigen Wölbungen der Blattoberfläche untersucht werden. Zur Untersuchung des Benetzungsverhaltens werden 5 µL Flüssigkeit mit einer Spritze durch eine PTFE-Kapillare (Typ N44; d = 0,776 mm) auf die Blattoberfläche getropft. Da auf der sehr rauen Oberfläche der Weinpflanzen keine genauen Randwinkelmessungen durchgeführt werden können, wird das Benetzungsverhalten qualitativ nach 0 s und 10 s beurteilt.

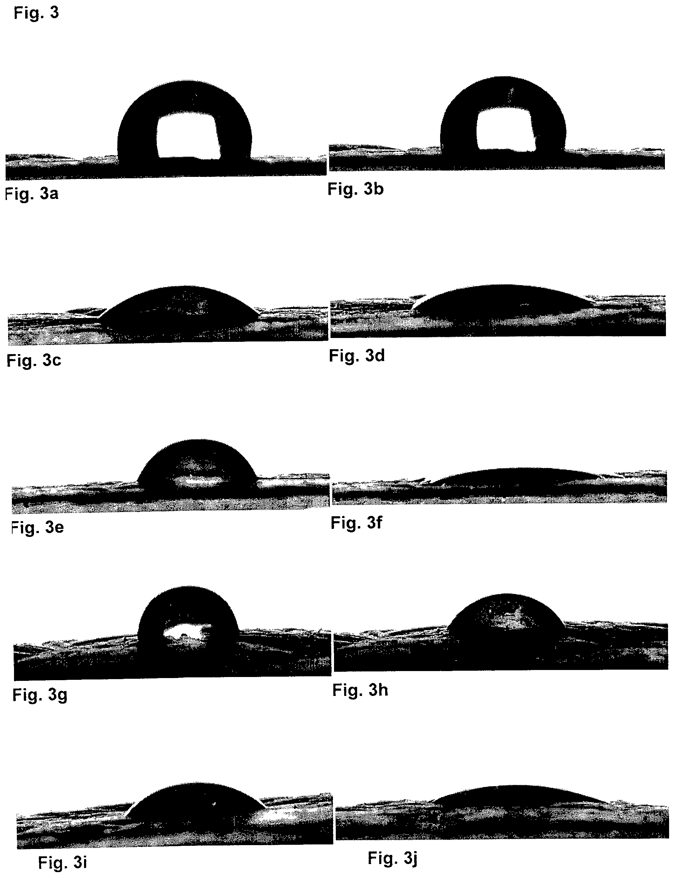

Abbildung 3 zeigt die Tropfen verschiedener wässriger Tensidlösungen im Vergleich zu reinem Wasser nach 0 s und nach 10 s auf den Weinblattoberflächen. Durch den Einsatz der verzweigten Fluortenside der Formel (III-2) und der Formel (III-1) verringert sich der Randwinkel der Tropfen gegenüber Wasser (H₂O) deutlich. Außerdem zeigt die Verbindung der Formel (III-2) bei der qualitativen Untersuchung nach 10 s bessere Benetzungseigenschaften als alle anderen Tensid Lösungen.

### Abbildungen

**Fig. 1** zeigt die Benetzung einer PTFE-Folie durch verschiedene wässrige Tensidlösungen im Vergleich zu reinem Wasser nach 0 s und nach 60 s. Es ist jeweils der Tropfenbasisdurchmesser d angegeben.
Fig. 1a: H₂O; t=0s: d= 3,8 mm
Fig. 1b**:** H₂O; t=60s:d= 3,8 mm
Fig. 1c: 0,1% der Verbindung der Formel (III-2); t=0s: d= 5,5 mm
Fig. 1d: 0,1% der Verbindung der Formel (III-2); t=60s: d= 12,3 mm
Fig. 1e: 0,1% der Verbindung der Formel (III-1); t=0s: d= 5,6 mm
Fig. 1f: 0,1 % der Verbindung der Formel (III-1); t=60s: d= 6,4 mm
Fig. 1g: 0,1% Fluortensid (linear C6); t=0s: d= 3,4 mm
Fig. 1h: 0,1% Fluortensid (linear C6); t=60s: d= 6,5 mm
Fig. 1i: 0,1% Gemini Tensid; t=0s: d= 5,0 mm
Fig. 1j: 0,1% Gemini Tensid; t=60s:d= 5,1 mm
**Fig. 2** zeigt den Tropfenbasisdurchmesser (V=20 µl; 0,1% Tensidkonzentration) auf PTFE-Folie nach verschiedenen Verweilzeiten
**Fig. 3** zeigt die Benetzung von Weinblattoberflächen durch verschiedene wässrige Tensidlösungen
Fig. 3a: H₂O; Tropfen bei t∼0s
Fig. 3b: H₂O; Tropfen bei t∼10s
Fig. 3c: 0,1% der Verbindung der Formel (III-1); Tropfen bei t∼0s
Fig. 3d: 0,1% der Verbindung der Formel (III-1); Tropfen bei t∼10s
Fig. 3e: 0,1% der Verbindung der Formel (III-2); Tropfen bei t∼0s
Fig. 3f: 0,1% der Verbindung der Formel (III-2); Tropfen bei t∼10s
Fig. 3g: 0,1% Fluortensid (linear C6); Tropfen bei t∼0s
Fig. 3h: 0,1 % Fluortensid (linear C6); Tropfen bei t∼10s
Fig. 3i: 0,1% Gemini Tensid; Tropfen bei t∼0s
Fig. 3j: 0,1% Gemini Tensid; Tropfen bei t∼10s

## Patentansprüche

1. Verwendung von Verbindungen der Formeln (III-1) und/oder (III-2) in Pestiziden, wobei * den jeweiligen Anknüpfungspunkt angibt

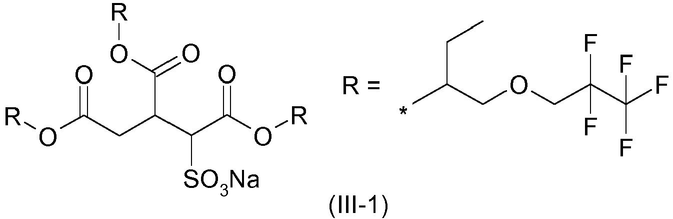

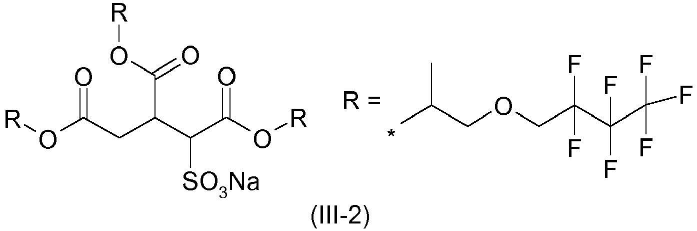

2. Verwendung gemäß Anspruch 1, **dadurch gekennzeichnet, dass** es sich bei den Pestiziden um Formulierungen handelt, die bei der Kultur von Nutz- oder Zierpflanzen über Spritzen aufgebracht werden.

3. Verwendung gemäß einem oder mehreren der Ansprüche 1 bis 2, **dadurch gekennzeichnet, dass** die Verbindungen der Formeln (III-1) und/oder (III-2) die Wirksamkeit der Pestizidwirkstoffe verbessern und/oder als Dispergiermittel, Emulsionsstabilisator und/oder Schauminhibitor wirken.

4. Pestizide enthaltend mindestens eine Verbindung der Formeln (III-1) und/oder (III-2), wobei * den jeweiligen Anknüpfungspunkt angibt

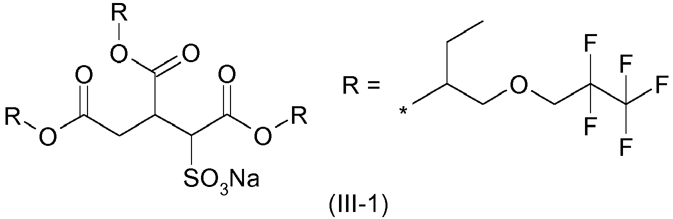

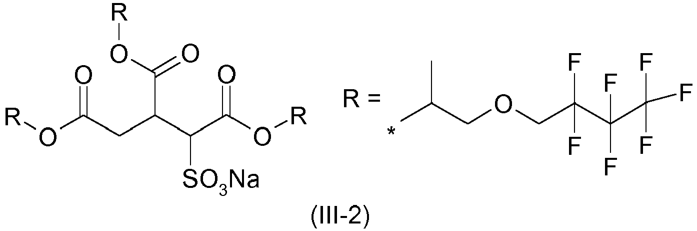

5. Pestizide gemäß Anspruch 4, **dadurch gekennzeichnet, dass** sie mindestens eine Verbindung der Formel (VI) enthalten
(RF-(Spacer)ₘ)ₙM (VI)
wobei
RF eine fluorhaltige Gruppe ist,
Spacer eine Einfachbindung oder eine organische funktionelle Kohlenstoffkette ist,
n ≥1 ist,
m = 0-1 und
M eine anionische, kationische, amphotere oder nicht-ionische Gruppe ist.

6. Pestizide gemäß einem oder mehreren der Ansprüche 4 bis 5, **dadurch gekennzeichnet, dass** in den Verbindungen der Formel (VI) die Gruppierung (RF-(Spacer)ₘ)ₙ- gleich C_{n'}F_{2n'+1}-, C_{n'}F_{2n'+1}-CH₂CH₂-, C_{n'}F_{2n'+1}-OCF₂CF₂-, C_{n'}F_{2n'+1}-OC₆H₄-, C_{n'}F_{2n'+1}-C(O)NH(CH₂)₃N=, C_{n'}F_{2n'+1}-SO₂NH(CH₂)₃N=, CF₃CCl₂(CF₂CFCl)_{n'-1}-CF₂- oder C₈F₁₇CH₂CH₂Si(CH₃)₂- ist, mit n' = 4-12, und M gleich -OPOO⁻ , -COO⁻, -SO₃⁻, -OSO₃⁻, -OP(O)(O⁻)O- oder -OP(O)O₂²⁻ ist, bevorzugt mit H⁺, Na⁺, K⁺, Li⁺ oder NH₄⁺ als Gegenion.

7. Pestizide gemäß einem oder mehreren der Ansprüche 4 bis 6, **dadurch gekennzeichnet, dass** sie mindestens eine Verbindung der Formel (VI) mit (RF-(Spacer)ₘ)ₙ- gleich C_{n'}F_{2n'+1}-CH₂CH₂- und M gleich -OPOO⁻, mit m = 1, n = 2 und n' = 4-12 und Na⁺ als Gegenion enthalten.

8. Pestizide gemäß einem oder mehreren der Ansprüche 4 bis 7, **dadurch gekennzeichnet, dass** sie mindestens eine Verbindung der Formeln (VII) bis (X) enthalten 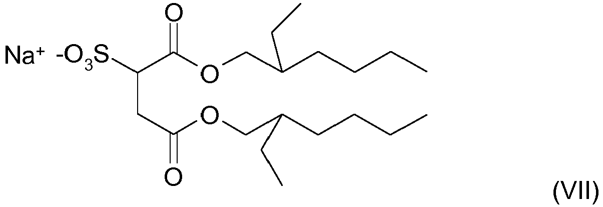 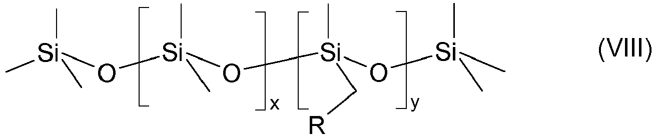 wobei x = 1-500, y = 1-500 und R = phenyl, methyl oder -(O-C₂H₃R')_{n"}-OR" ist, mit n" = 1-1000, R' = lineare und verzweigte Alkylrest und R" = lineare und verzeigte Alkylreste,
M₂D'(E_{n"'}P) (IX)
wobei M = (CH₃)₃SiO-, D' = Si(R"'), E = -OCH₂CH₂, n'" = 5 - 40 und P = -OH, -OMe, oder -OAc ist, mit R'" = lineare und/oder verzweigte Alkylkette, 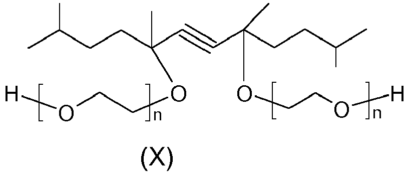 wobei n = 1-100 ist.

9. Pestizide gemäß einem oder mehreren der Ansprüche 4 bis 8, **dadurch gekennzeichnet, dass** es sich um flüssige Pestizidformulierungen handelt, die mittels Spritz- oder Sprühverfahren angewendet werden.

10. Pestizide gemäß einem oder mehreren der Ansprüche 4 bis 9, **dadurch gekennzeichnet, dass** es sich bei den Pestiziden um Pflanzenschutzmittel handelt.

## Claims

1. Use of compounds of the formulae (III-1) and/or (III-2) in pesticides, where * indicates the respective linking point

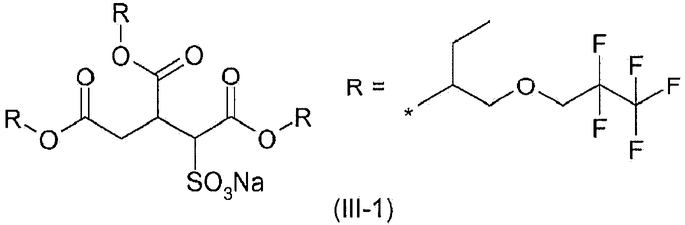

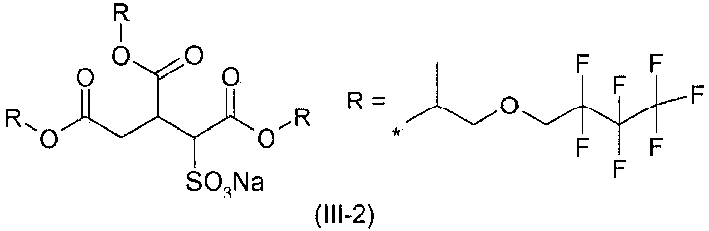

2. Use according to Claim 1, **characterised in that** the pesticides are formulations which are applied by spraying in the cultivation of crop or ornamental plants.

3. Use according to one or more of Claims 1 to 2, **characterised in that** the compounds of the formulae (III-1) and/or (III-2) improve the efficacy of the pesticide active compounds and/or act as dispersant, emulsion stabiliser and/or foam inhibitor.

4. Pesticides comprising at least one compound of the formulae (111-1) and/or (III-2), where * indicates the respective linking point

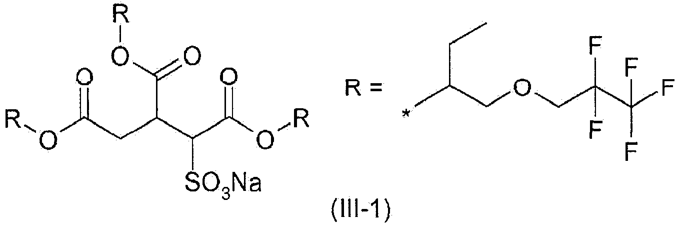

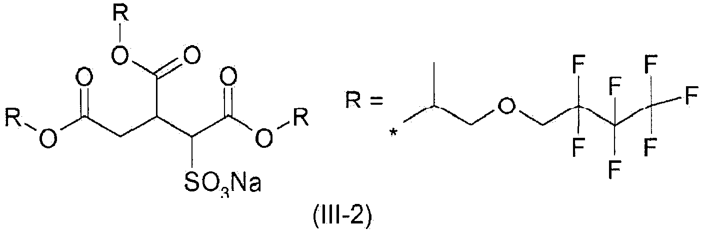

5. Pesticides according to Claim 4, **characterised in that** they comprise at least one compound of the formula (VI)
(RF-(spacer)ₘ)ₙM (VI)
where
RF is a fluorine-containing group,
spacer is a single bond or an organic functional carbon chain,
n is ≥1,
m = 0-1 and
M is an anionic, cationic, amphoteric or non-ionic group.

6. Pesticides according to one or more of Claims 4 to 5, **characterised in that** the group (RF-(spacer)ₘ)ₙ- in the compounds of the formula (VI) is equal to C_{n'}F_{2n'+1}-, C_{n'}F_{2n'+1}-CH₂CH₂-, C_{n'}F_{2n'+1}-OCF₂CF₂-, C_{n'}F_{2n'+1}-OC₆H₄-, C_{n'}F₂ₙ₊₁-C(O)NH(CH₂)₃N=, C_{n'}F_{2n'+1}-SO₂NH-(CH₂)₃N=, CF₃CCl₂(CF₂CFCl)_{n'-1}-CF₂- or C₈F₁₇CH₂CH₂Si(CH₃)₂-, where n' = 4-12, and M is equal to -OPOO⁻, -COO⁻, -SO₃⁻, -OSO₃⁻, -OP(O)(O⁻)O- or -OP(O)O₂²⁻, preferably with H⁺, Na⁺, K⁺, Li⁺ or NH₄⁺ as counterion.

7. Pesticides according to one or more of Claims 4 to 6, **characterised in that** they comprise at least one compound of the formula (VI) where (RF-(spacer)m)n- is equal to C_{n'}F_{2n'+1}-CH₂CH₂- and M is equal to -OPOO⁻, where m = 1, n = 2 and n' = 4-12 and with Na⁺ as counterion.

8. Pesticides according to one or more of Claims 4 to 7, **characterised in that** they comprise at least one compound of the formulae (VII) to (X) 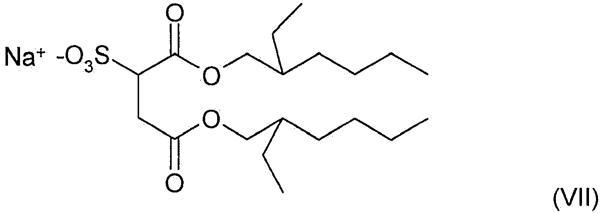 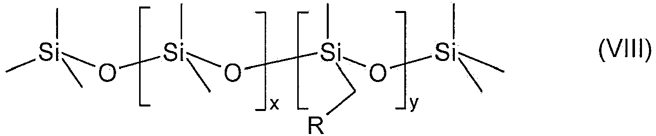 where x = 1-500, y = 1-500 and R = phenyl, methyl or -(O-C₂H₃R')_{n"}-OR", where n" = 1-1000, R' = linear and branched alkyl radical and R" = linear and branched alkyl radicals,
M₂D'(E_{n"'}P) (IX)
where M = (CH₃)₃SiO-, D' = Si(R"'), E = -OCH₂CH₂, n'" = 5 - 40 and P = -OH, -OMe or -OAc, where R"' = linear and/or branched alkyl chain, 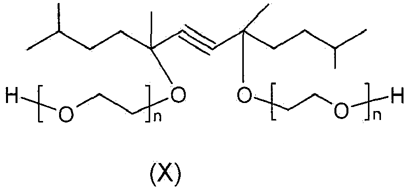 where n = 1-100.

9. Pesticides according to one or more of Claims 4 to 8, **characterised in that** they are liquid pesticide formulations which are applied by means of spraying methods.

10. Pesticides according to one or more of Claims 4 to 9, **characterised in that** the pesticides are crop protection agents.

## Revendications

1. Utilisation de composés de formules (III-1) et/ou (III-2) dans des pesticides, où * indique le point de liaison respectif

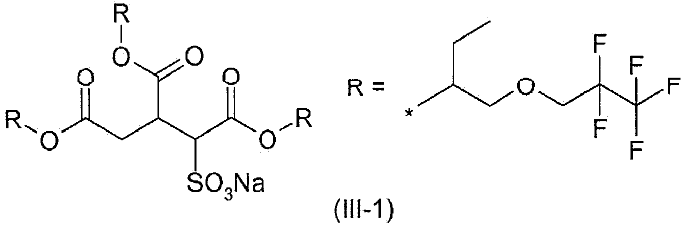

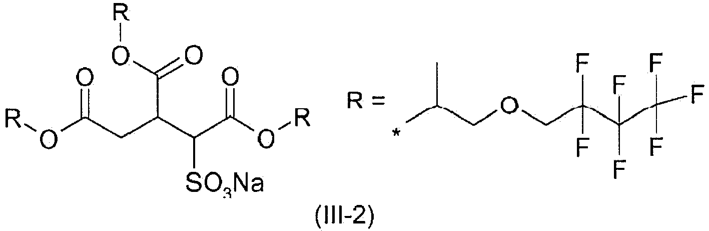

2. Utilisation selon la revendication 1, **caractérisée en ce que** les pesticides sont des formulations qui sont appliquées par pulvérisation dans la culture de plantes ornementales ou de culture.

3. Utilisation selon l'une ou plusieurs parmi les revendications 1 à 2, **caractérisée en ce que** les composés de formules (III-1) et/ou (III-2) améliorent l'efficacité des composés actifs pesticides et/ou ont un rôle de dispersant, de stabilisant d'émulsion et/ou d'agent anti-mousse.

4. Pesticides comprenant au moins un composé de formules (III-1) et/ou (III-2), où * indique le point de liaison respectif

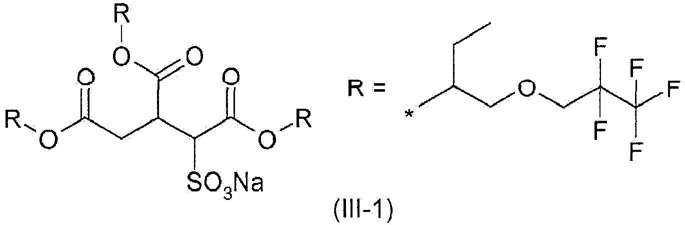

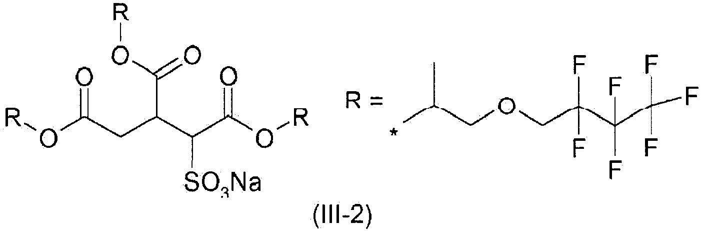

5. Pesticides selon la revendication 4, **caractérisés en ce qu'**ils comprennent au moins un composé de formule (VI)
(RF-(espaceur)ₘ)ₙM (VI)
où
RF est un groupement fluoré,
l'espaceur est une liaison simple ou une chaîne carbonée organique fonctionnelle,
n est ≥1,
m = 0-1 et
M est un groupement anionique, cationique, amphotère ou non ionique.

6. Pesticides selon l'une ou plusieurs parmi les revendications 4 à 5, **caractérisés en ce que** le groupement (RF-(espaceur)m)n- dans les composés de formule (VI) est égal à C_{n'}F_{2n'+1}-, C_{n'}F_{2n'+1}-CH₂CH₂-, C_{n'}F_{2n'+1}-OCF₂CF₂-, C_{n'}F_{2n'+1}-OC₆H₄-, C_{n'}F_{2n'+1}-C(O)NH(CH₂)₃N=, C_{n'}F_{2n'+1}-SO₂NH(CH₂)₃N=, CF₃CCl₂(CF₂CFCl)_{n'-1}-CF₂- ou C₈F₁₇CH₂CH₂Si(CH₃)₂-, où n' = 4-12, et M est égal à -OPOO⁻, -COO⁻, -SO₃⁻, -OSO₃⁻, -OP(O)(O⁻)O- ou -OP(O)O₂²⁻, préférablement avec H⁺, Na⁺, K⁺, Li⁺ ou NH₄⁺ comme contre-ion.

7. Pesticides selon l'une ou plusieurs parmi les revendications 4 à 6, **caractérisés en ce qu'**ils comprennent au moins un composé de formule (VI) où (RF-(espaceur)m)n- est égal à C_{n'}F_{2n'+1}-CH₂CH₂- et M est égal à -OPOO⁻, où m = 1, n = 2 et n' = 4-12 et avec Na⁺ comme contre-ion.

8. Pesticides selon l'une ou plusieurs parmi les revendications 4 à 7, **caractérisés en ce qu'**ils comprennent au moins un composé de formules (VII) à (X) 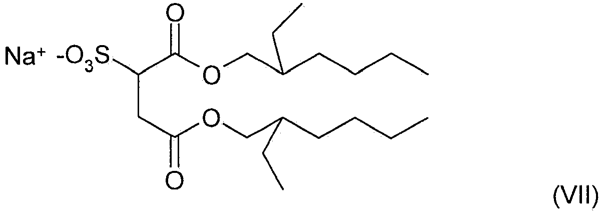 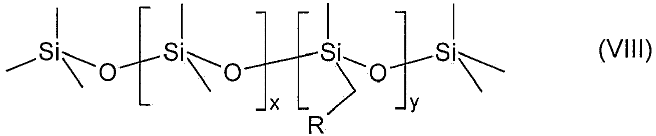 où x = 1-500, y = 1-500 et R = phényle, méthyle ou -(O-C₂H₃R')_{n"}-OR", où n" = 1-1000, R' = un radical alkyle linéaire et ramifié et R" = des radicaux alkyle linéaires et ramifiés,
M₂D'(E_{n"'}P) (IX)
où M = (CH₃)₃SiO-, D' = Si(R"'), E = -OCH₂CH₂, n"' = 5 - 40 et P = -OH, -OMe ou -OAc, où R'" = une chaîne alkyle linéaire et/ou ramifiée, 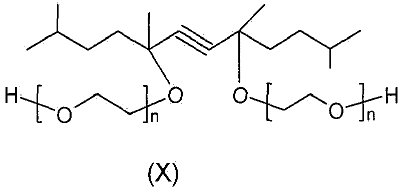 où n = 1-100.

9. Pesticides selon l'une ou plusieurs parmi les revendications 4 à 8, **caractérisés en ce qu'**il s'agit de formulations pesticides liquides qui sont appliquées au moyen de méthodes de pulvérisation.

10. Pesticides selon l'une ou plusieurs parmi les revendications 4 à 9, **caractérisés en ce que** les pesticides sont des agents de protection des cultures.
